# EUROPEAN PATENT SPECIFICATION

(11) **EP 4 093 372 B1**
(45) Date of publication and mention of the grant of the patent: **03.04.2024**
(21) Application number: 20708605.9
(22) Date of filing: 23.01.2020
(51) Int. Cl.: A61K 9/00, A61K 31/07, A61P 27/04, A61K 36/02, A61K 31/593, A61K 31/23, A61K 31/202, A61K 9/127, A61K 9/10, A61K 36/06

(54) **OPHTHALMIC COMPOSITION FOR THE TREATMENT OF DRY EYE DISEASE**
OPHTHALMISCHE ZUSAMMENSETZUNG ZUR BEHANDLUNG DES TROCKENEN AUGES
COMPOSITION OPHTALMIQUE POUR LE TRAITEMENT DE LA SÉCHERESSE OCULAIRE

(43) Date of publication of application: 30.11.2022
(73) Proprietor: Visufarma S.p.A., 00136 Roma (IT)
(72) Inventor: BIGIONI, Anna Rita, 00189 Roma (IT)
(74) Representative: Di Giovine, Paolo
(86) International application number: PCT/IB2020/050533
(87) International publication number: WO 2021/148845

(56) References cited:
- WO-A1-2012/059158
- WO-A1-2014/035450
- US-A- 6 162 801
- US-A1- 2006 009 522
- US-A1- 2014 024 625

## Description

The present invention relates to an ophthalmic composition comprising omega-3 fatty acids, Vitamin D3, Vitamin A or esters and liposomes thereof and to the methods for the preparation thereof. The present invention further relates to the use of such composition for preventing and treating dry eye disease (DED), in particular in patients undergoing surgical procedures such as cataract or the like.

### STATE OF PRIOR ART

Dry eye disease (in short DED) is an extremely frequent clinical condition affecting from 14 to 33% of population. "Dry eye" is the general term to designate abnormalities in precorneal tear film; the occurrence mechanisms may be the decrease in the tear production, the increase in the evaporation of tear film or (more frequently) the combination of the previous two. The term DED is used for all causes of ocular dryness, including immune forms (dry keratoconjunctivitis, KCS); dry eye linked to age; dry eye consequent to corneal lesions, infections, disturbances or nutritional deficiencies (including vitamins); side effects of drugs used by systemic route (for example diuretics or benzodiazepine) or by ocular topical route (for example eye drops for the treatment of glaucoma or chronic use of eye drops with preservatives); intolerance to contact lenses; ocular stress and gland and tissue destruction; environmental exposure to smog, smoke, excessively dry air, particles suspended in air. Moreover, it is known that dry eye disease is induced or exacerbated by the ocular surgical procedures (Gomes JAP, Azar DT, Baudouin C, et al. TFOS DEWS II iatrogenic report. Ocul Surf. 2017;15(3):511-538. doi:10.1016/j.jtos.2017.05.004). This aspect is particularly important in cataract surgery, considering the frequency of such pathology. Cataract surgery causes DED in about 10% of population (Kasetsuwan N,et al. PLoS One 2013;8(11):e 78657); DED is induced independently from the used technique (for example phacoemulsification by traditional route or associated to femtolaser, SICS and ECCE). Moreover, the presence of DED before cataract surgery constitutes a particularly relevant clinical problem since it can be associated to refractive error (wrong calculation of the value of the lens to be implanted in the eye during operation) (Chuang J, Shih KC, Chan TC, Wan KH, Jhanji V, Tong L. Preoperative optimization of ocular surface disease before cataract surgery. J Cataract Refract Surg. 2017;43(12):1596-1607.doi:10.1016/j.jcrs.2017.10.033), to worsening of severity of DED itself in post-surgery and to occurrence of chronic post-surgery pain (Gomes JAP, Azar DT, Baudouin C, et al. TFOS DEWS II iatrogenic report. Ocul Surf. 2017;15(3):511-538. doi:10.1016/j.jtos.2017.05.004). Then, it is crucial to recognize and treat DED before performing ocular surgery with the purpose of bringing the patient to surgery with a healthy ocular surface. The surface preparation is essential in patients with ocular disease of the ascertained surface, and it is also useful in those with minimum signs or symptoms of ocular surface disease. DED study can be performed with several examinations to investigate both clinical signs and symptoms. Thereamong, the most used ones are tear break-up time (TBUT or BUT), which, after staining with sodium fluorescein, measures the tear film break-up time elapsing after blinking; Schirmer test, to evaluate the amount of tear produced by the ocular surface in 5 minutes; staining of ocular surface with fluorescein, expressing the presence of corneal or conjunctival epitheliopathy consequent to DED; at last, questionnaires for the measurement of DED symptoms can be used; thereamong, Ocular Surface Disease Index (OSDI) is one of the most widespread. The treatment of dry eye disease is mainly limited to the administration of artificial tears (saline solution, carbomers, lipids) or anti-inflammatory agents (steroids, cyclosporin, less frequently nonsteroidal anti-inflammatory drugs). The patent EP 2 408 426 B1 describes an ophthalmic composition comprising as active ingredients polyunsaturated fatty acids of the omega-3 and omega-6 types, formulated in a stable composition in hydrogel, for topical use in the prevention or treatment of dry eye disease. WO 2014/035450 A1 discloses ophthalmic compositions that include omega-3 fatty acids for alleviating symptoms associated with dry eye and may further include vitamin A. US 2006/009522 A1 discloses ophthalmic compositions comprising at least one omega-6 fatty acid and at least one omega-3 fatty acid for use in treating dry eye that may be administered by on-site delivery using liposomes.

In the state of art, the need for providing ophthalmic compositions for topical use in the prevention or treatment of dry eye disease is still much felt.

### SUMMARY OF THE INVENTION

Clinical studies performed by the present inventors have demonstrated that the topical administration of an ophthalmic composition comprising as active principles omega-3 fatty acids, Vitamin D3, Vitamin A or esters thereof and liposomes as carriers is able to prevent and treat effectively dry eye disease. Such studies were performed on different classes of patients, in particular on patients undergoing cataract surgery. The clinical studies are shown in greater detail in the experimental section of the present description.

A first objective of the present invention is an ophthalmic composition comprising omega-3 fatty acids, Vitamin A or esters thereof, Vitamin D3 and liposomes.

An additional objective of the present invention is such composition for use in the prevention and/or treatment of dry eye disease, in particular associated to a cataract surgery.

An additional objective is a method for preparing such composition comprising the mixing of the active principles optionally with one or more excipients.

The advantages, features and use modes of the present invention will result evident from the following detailed description of some embodiments, shown by way of example and not for limitative purposes.

### BRIEF DESCRIPTION OF FIGURES

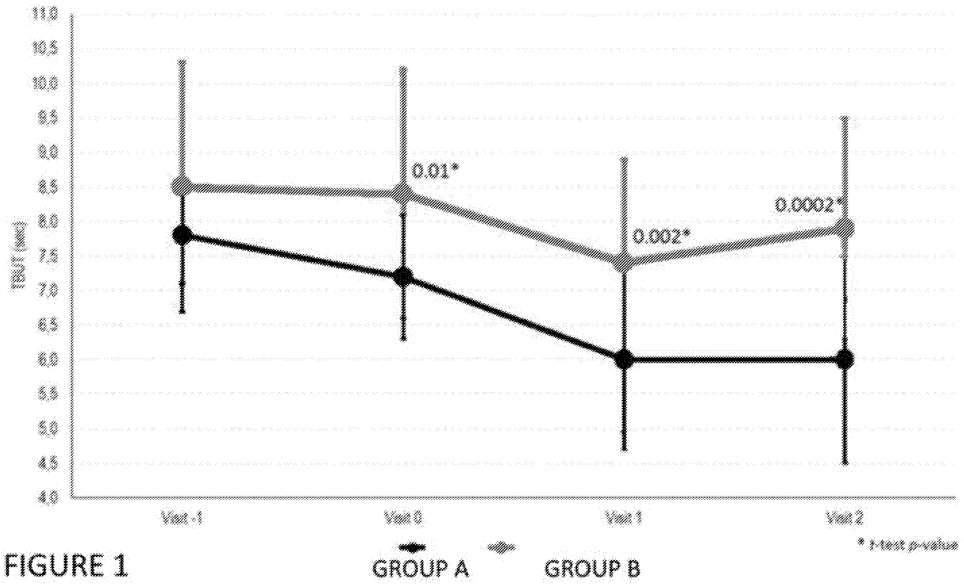

Figure 1. Trend of TBUT (seconds) among study visits by study treatment group.
   The graph shows TBUT mean values at each visit (points) and the standard deviation (bars). The two study groups are reported: Group A, patients subjected to the treatment with the ophthalmic composition according to a preferred embodiment of the present invention three times a day starting from end of visit -1 and throughout the duration of the study; Group B, control patients who did not receive any tear substitute. The patients were randomized to one of the two groups at the beginning of the study. The study included 4 visits: baseline (Visit -1), cataract surgery day (Visit 0), 1 week (Visit 1), 1 month (Visit 2).
   From Figure 1 it can be seen that the two groups had superimposable TBUT at visit -1, while then statistically significant and clinically relevant differences were obtained to the advantage of Group A for the rest of the study. The herein described composition allowed to mitigate the dangerous effect of the cataract surgery on DED throughout the whole study; in fact TBUT in group A decreased on the average, from the beginning and the end of the study, only by 0.6 sec, while in group B such difference was 1.6 sec (P=0.0002 at the end of the study).
   The treatment with the herein described composition allowed the patients to arrive at the surgery with a superimposable TBUT at visit of beginning of the study (8.4 ±1.8 sec vs 8.5±1.8); vice versa, in the control group a decrease in TBUT from 7.8 ± 0.7 to 7.2 ± 0.9 sec was noted. The treatment with the herein described composition then allowed to perform operations on healthier ocular surfaces, and then more resistant to the potential risks induced by surgery.
   At last, TBUT values lower than 7 sec are unanimously recognized as representative of DED; the mean values at the various visits in group A have always been higher than this cut-off, while in group B the last two visits usually fell below this value, by confirming the usefulness of the present invention in protecting the patients from the risk of incurring in DED after cataract surgery.

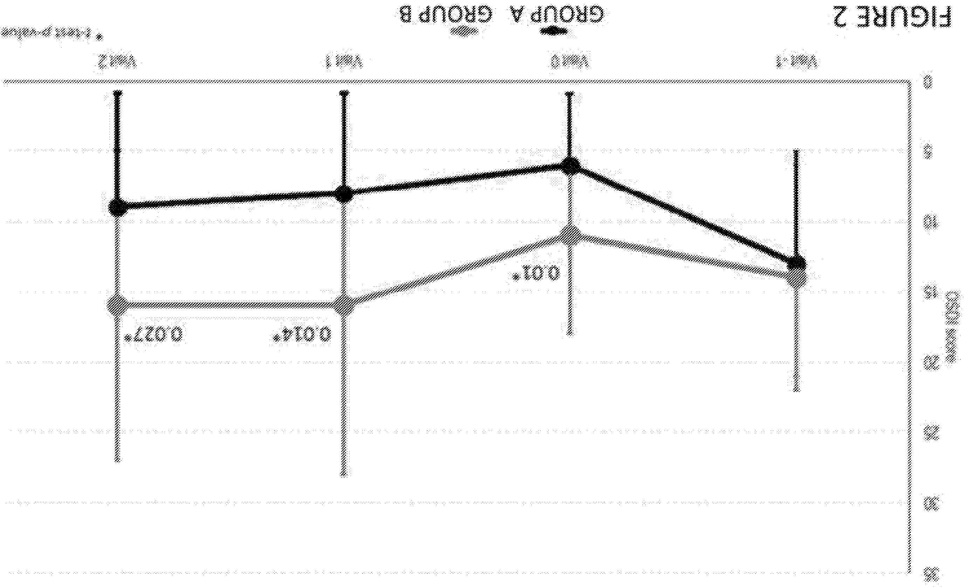

Figure 2. Trend of OSDI Questionnaire Score among study visits by study treatment group.
   Analogously to Figure 1, this figure shows OSDI score in the 4 visits for the two study groups. At visit -1, the two groups had similar OSDI values. The treatment with the herein described composition allowed the patients to arrive at surgery with significantly lower ocular discomfort symptoms compared to the control group (almost halved numeral data: 6 ± 5 vs 11 ± 7, P=0.01). The difference between the two groups further increased at the subsequent two visits, with statistically significant differences.

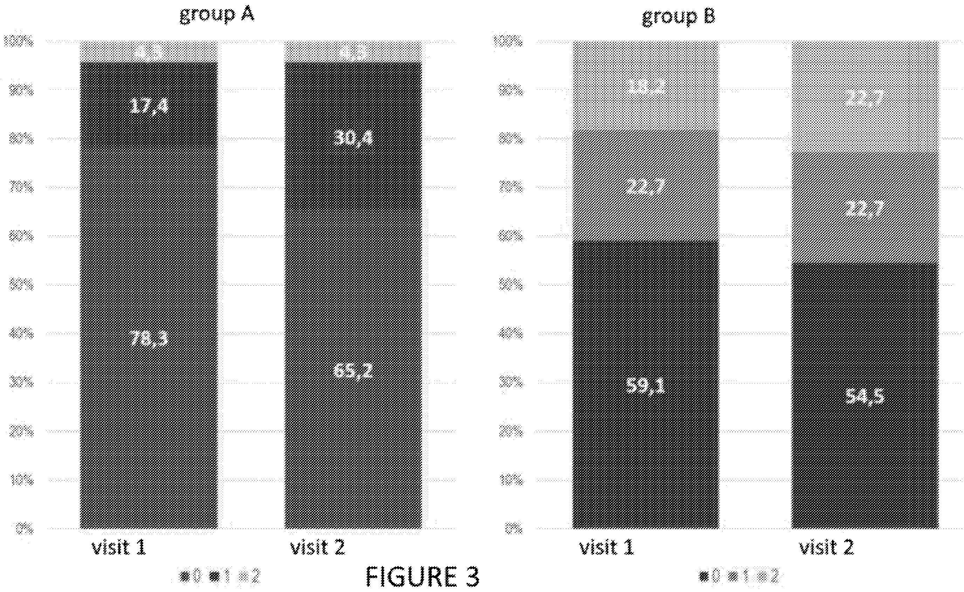

Figure 3. Distribution of patients assessed by staining grade with fluorescein by study group at the post-surgery Visit 1 and Visit 2.
   This figure aims at comparing the two groups in post-surgery visits as to staining of the ocular surface with fluorescein, which, if positive, shows alteration in epithelial barrier. Staining absence was more frequent in group A (treatment with composition the present invention relates to) at each visit: 78 and 65% compared to 59 and 54% for group B. Moreover, the percentage of moderate staining (level 2) was 4% in group A, compared to 18%-23% in group B. This figure summarises the evidence that the use of patent treatment is most associated to a healthy ocular surface in post-surgery with respect to control. It is to be noted that the presence of corneal epitheliopathy after cataract surgery is one of the risk factors for endophtalmitis, fearsome infective complication due to penetration and proliferation of pathogen germs inside the eye.

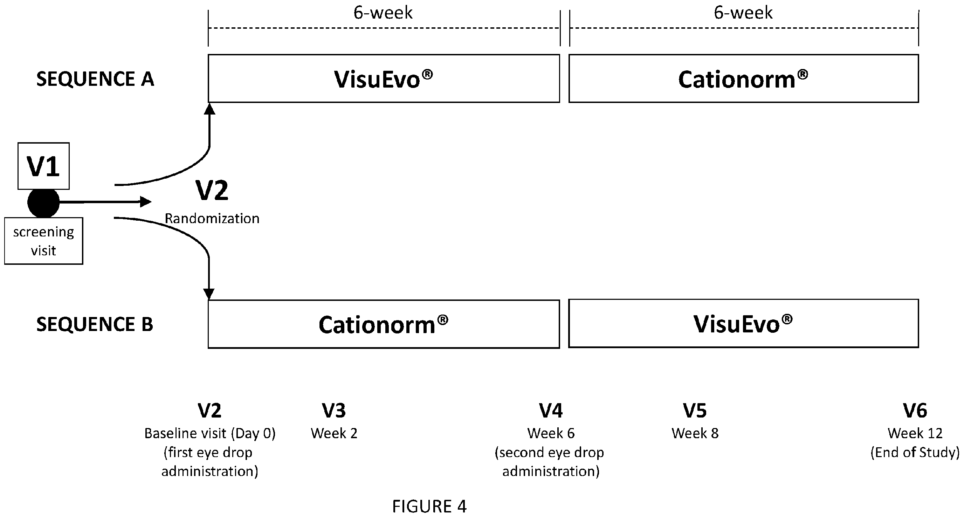

   In figures 4-11 the composition according to the preferred embodiment of the present invention described in detail in example 1 is designated as Visuevo^{®}.
Figure 4. Scheme of the second study, performed on patients with DED, con crossover drawing and comparison between VisuEvo and Cationorm (one of the reference drugs for the symptomatic treatment of DED). The figure shows that patients, after baseline, received 1:1 randomization at two treatment arms with opposite sequences. The study consisted of 6 visits.

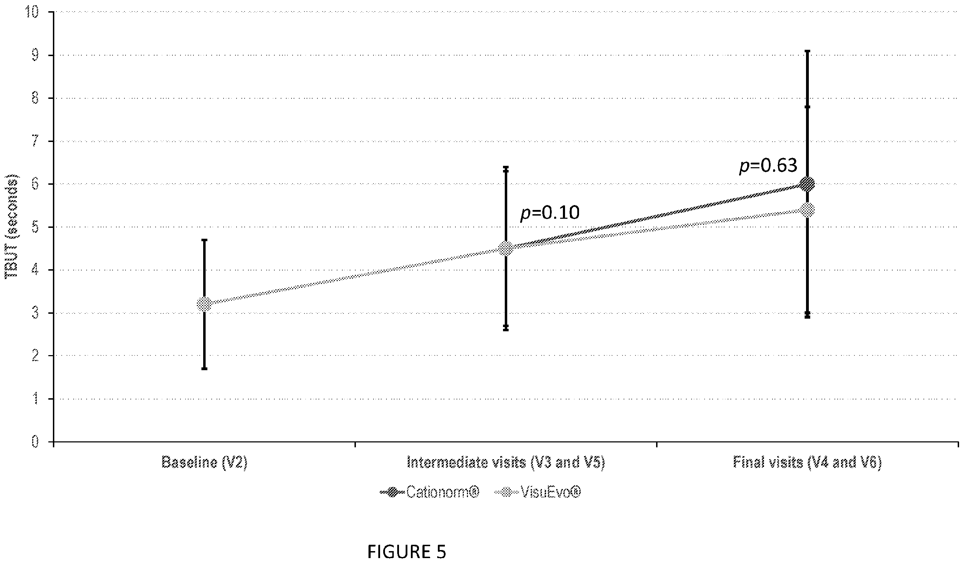

Figure 5. Trend of TBUT during the study. This figure evaluates the whole treatment periods for each drug (independently from crossover sequence). The figure shows that both products improve the main parameter under study, then both having an adequate clinical effectiveness, without statistically nor clinically significant differences between the two products. This figure justifies non-inferiority of VisuEvo on Cationorm.

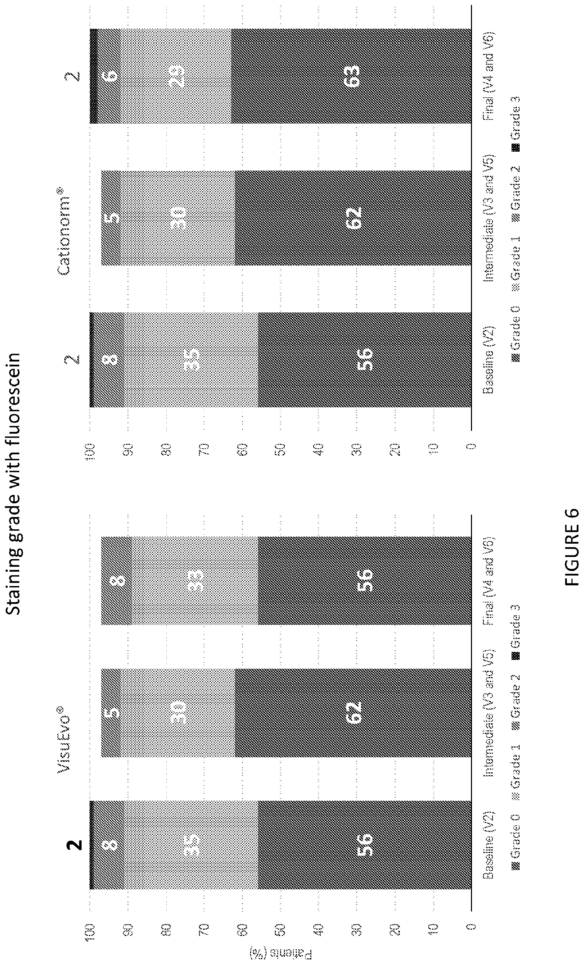

Figure 6. The figure represents the distribution of staining with fluorescein by stages during the several study visits: one more time, the two products obtain superimposable performances.

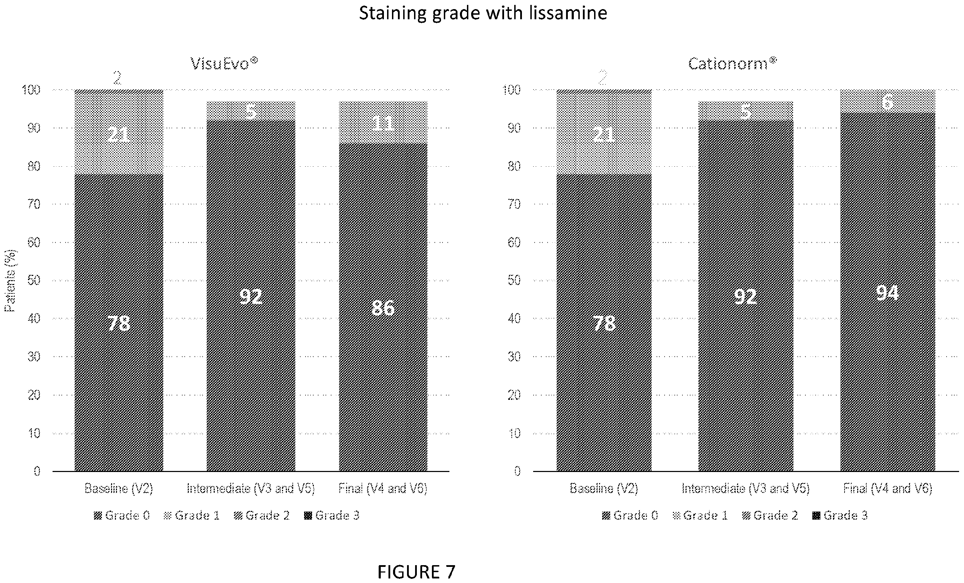

Figure 7. The figure represents the distribution of staining with lissamine by stages during the several study visits. The two products obtain superimposable performances.

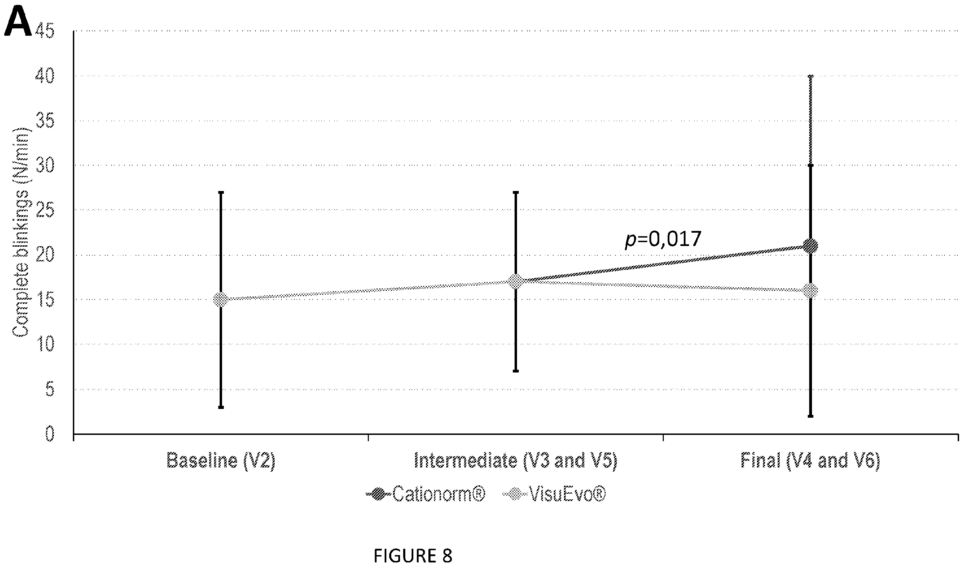

Figure 8. This figure shows that, in front of superimposable data at baseline and intermediate visits, in the conclusive visits VisuEvo was associated to a significantly lower number of complete blinking per minute with respect to competitor.

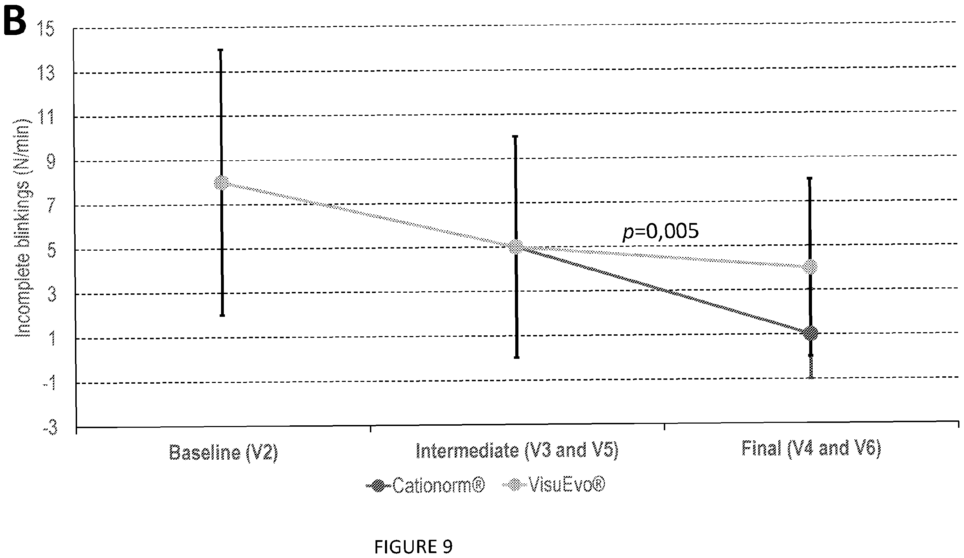

Figure 9. The number of incomplete blinking per minute, as the total ones, is expression of DED severity. In this slide the most important piece of data is the progressive reduction in the number of incomplete blinking during the study with both products; the reduction with VisuEvo is less marked than the one obtained by Cationorm.

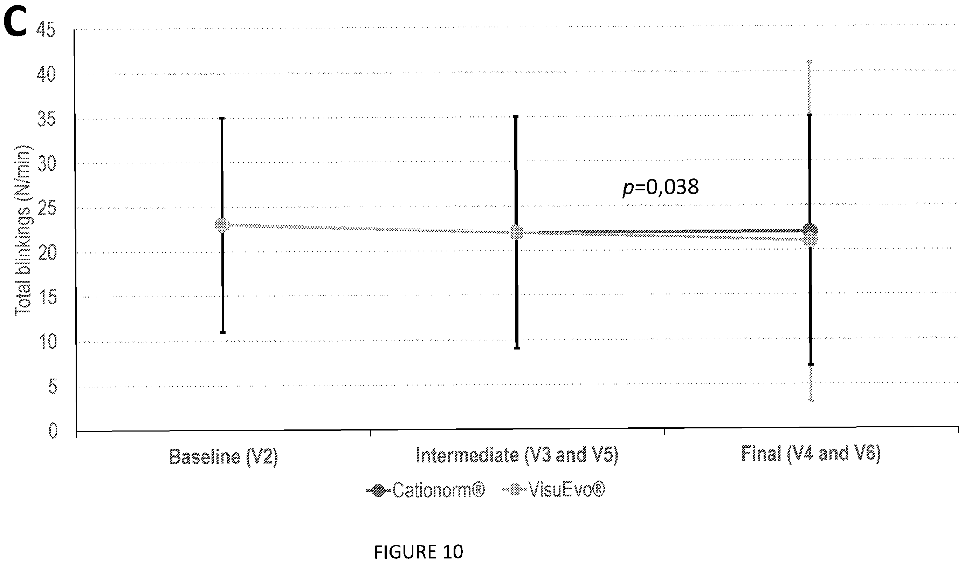

Figure 10. The number of total (complete + incomplete) blinking per minute expresses the need for ocular surface to re-distribute the tear film on its surface. Ideally, the number of total blinking decreases if the ocular surface is healthier (in subject without dry eye, the number of blinking per minute is lower than 10). In this graph, it is demonstrated that, in front of superimposable data at baseline and intermediate visits, in the conclusive visits VisuEvo was associated to a significantly lower number of blinking per minute with respect to competitor, by demonstrating a clinical superiority.

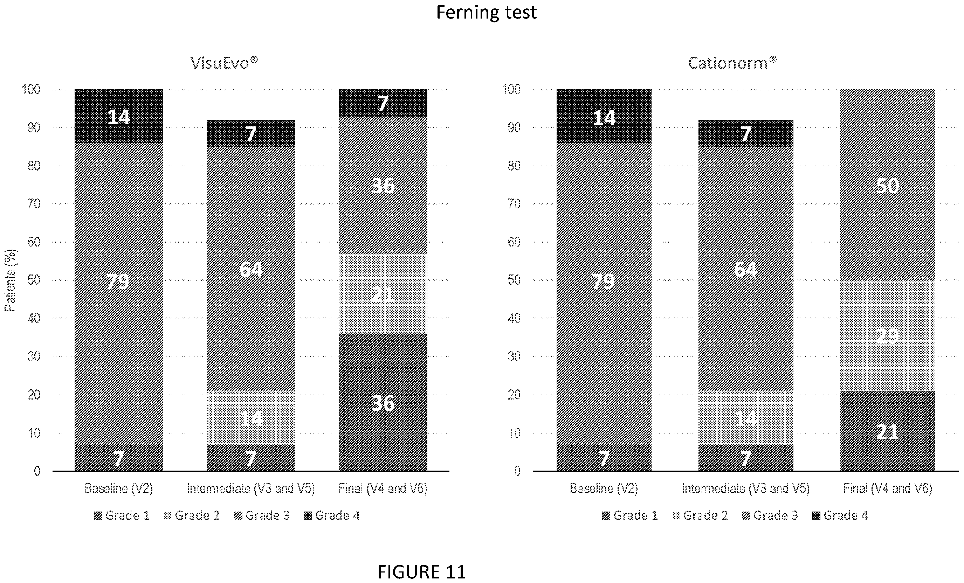

Figure 11. Distribution of ferning test during the study in the two groups. In the intermediate visits data are superimposable, while at last visit the percentage of subjects with grades 1-2 (considered normal in literature) is 57 with the product Visuevo vs 50% with the competitor. VisuEvo then seems to have a higher clinical effectiveness in this sub-group of patients.

### DETAILED DESCRIPTION OF THE INVENTION

The present invention relates to an ophthalmic composition comprising omega-3 fatty acids, Vitamin D3, Vitamin A or esters thereof and liposomes.

Preferably the composition will include omega-3 fatty acids of vegetable or algal origin, for example such omega-3 fatty acids will be eicosapentaenoic acid (EPA), docosapentaenoic acid (DPA) and/or docosaesaenoic acid (DHA). According to a preferred embodiment Omega-3 oils derived from *Schizochytrium sp* marine microalga will be used.

Vitamin D3, also called cholecalciferol, is a vitamin of group D with the following structure:

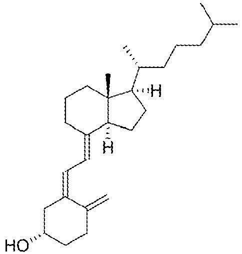

The composition according to the present invention will include Vitamin A, esters thereof or mixtures thereof. Under Vitamin A both retinol and analogues thereof, called retinoids, are meant, Vitamin A appears in three different forms: alcoholic (retinol), aldehyde (retinaldehyde) and acid (retinoic acid). In a preferred embodiment in the composition Vitamin A palmitate, also known as palmitate retinyl, will be used.

The composition according to the present invention will further include liposomes. In the present description under the term liposomes phospholipidic vesicles having nanometric sizes are meant, preferably such liposomes consist of a double phospholipidic layer. According to an embodiment one or more of the active ingredients of the composition according to the present description are incorporated inside the liposomes so as to be suitably vehiculated. According to an embodiment the sizes of liposomes will be preferably comprised between 75 and 0300 nm. According to a preferred embodiment such liposomes will consist of a double phospholipidic layer, for example hydrogenated phospholipids. Examples of suitable liposomes available on the market are Phospholipids Lipoid S80.

In the present description under the term ophthalmic composition a composition suitable for the topical administration on the ocular surface is meant. The person skilled in the art could select which excipients and/or diluents are ophthalmologically compatible. Such compositions could obviously further include one or more carriers, diluents and/or excipients suitable for the preparation of ophthalmic compositions, for example PEG400. All carriers, diluents or excipients tolerated by the eye are suitable for the preparation of ophthalmic compositions.

The topical formulation optionally includes a preservative, such as for example benzalkonium chloride or N-hydroxymethylglycinate or PHMB or other. The formulation pH could be adjusted by adding possible acids, bases or buffer systems for adjusting pH, physiologically and ophthalmologically acceptable, preferably in the range from about 6.0 to 8.0; Examples of acids include acetic, boric, citric, lactic, phosphoric, hydrochloric acids and the like, and examples of bases include sodium hydroxide, potassium hydroxide, and the like. Buffer systems include citrate, phosphate, borate, carbonate and mixtures of the above-mentioned acids, bases and buffer systems. Said compositions for topical use could be in any form considered suitable by the person skilled in the art to be applied directly on the ocular surface such as for example solution, ointment, suspension, eye drop, gel, cream, foam, spray, pomade. For example, the eye drop can include salts such as monobasic sodium phosphate, dibasic sodium phosphate, sodium chloride or a combination thereof to obtain physiological pH and osmolality (pH 6.0-8.0 and 200-400 mOsm/Kg). Preferably it can be a physiological solution with 0.9% NaCl.

The ophthalmic composition of the present invention could be used in the prevention and treatment of all different forms of dry eye disease (in short DED), for example dry keratoconjunctivitis (KCS), dry eye linked to age, corneal lesion, infections, disturbances or nutritional deficiencies (including vitamins), pharmacological side effects of drugs, intolerance to contact lenses, ocular stress and gland and tissue destruction, environmental exposure to smog, smoke, excessively dry air, particles suspended in air, autoimmune diseases, etc.

In particular the ophthalmic composition of the present invention could be used in the prevention and treatment of dry eye disease associated to a cataract surgery, independently from the used specific surgical procedure. According to an embodiment the composition will be administered according to a dosing regimen including an administration one or several times per day, preferably three, for at least four weeks, two weeks before the cataract surgery and two weeks after surgery.

As described in greater detail in the experimental section, the ophthalmic composition of the present invention resulted to be effective even in the treatment of specific classes of patients with DED, in particular patients with high levels of evaporation, menopause women, independently from the use of hormone integration, patients with active obstructive Meibomian gland dysfunction and glaucomatous patients.

The ophthalmic composition according to the present invention could include the above-described substances in the following preferred ranges, expressed as percentage in weight w/w:
Omega 3 fatty acids comprised between 0.01 and 2%, preferably between 0.1 and 0.5%; and/or
Vitamin A palmitate comprised between 0.01 and 2%, preferably between 0.02 and 0.5%; and/or
Vitamin D3 comprised between 0.01 and 2%, preferably between 0.02 and 0.5%; and/or
Liposomes comprised between 0.5 and 4%, preferably between 0.5 and 2%.

The present description further provides a method for the treatment of DED comprising the administration of effective amounts of a composition, as herein described, to patients requiring it. The exact dosage and the administration frequency of the compositions will depend upon the particular severity of the condition to be treated, upon age, weight and general physical conditions of the particular patient as it is well known to the persons skilled in the art.

The invention is described in details hereinafter in the following examples by way of example only, without limiting the conferred protection scope.

### EXAMPLES

### EXAMPLE 1.

The eye drop formulation used in clinical experiments is shown hereinafter, the amounts of the single components are shown in grams per each 100 grams of solution:

| Component | %w/w |
|---|---|
| Purified water until | 100 g |
| Phospholipids Lipoid S80 | 1.00 g |
| Omega 3 | 0.20 g |
| Vitamin A palmitate | 0.04 g |
| Vitamin D | 0.04 g |
| Boric acid | 0.776 g |
| Sodium tetraborate | 0.184 g |
| PEG 400 | 0.10 g |
| EDTA disodium dihydrate | 0.10 g |
| Sodium chloride | 0.425g |

### EXAMPLE 2 PREPARATION OF THE COMPOSITION OF EXAMPLE 1

### Collection and weighing of raw materials

The compliant raw materials are collected from the raw-material warehouse and weighed on the scale. The weighing and the batch of each raw material is shown on the production Batch Record di.

### Preparation of the Oil Solution

An oil solution is prepared by dissolving Vitamin A palmitate and Vitamin D in Omega 3 oil.

### Preparation of the Borate Buffer

In a steel tank, positioned on a yoke, a portion of depurated water is added and, in sequence, the following raw materials are added: Boric acid, Sodium Tetraborate dihydrate, EDTA and Sodium chloride. The remaining depurated water is added until obtaining the desired weight.

### Preparation of the Liposomal formulate

The phospholipids and the oil solution are added to a suitable amount of borate buffer and emulsified to obtain a liposomal formulation, the particle sizes thereof are reduced through a high-pressure extrusion process (1400 bar).

### Filtration and sterilization

Sterilization by filtration takes place by using a sterile single-use custom bag (Allegro System) equipped with a sterile 0.2-µm filter. The Allegro System is equipped with a patented connection/disconnection apparatus (Kleenpak) which guarantees to keep sterility of the bag and the content thereof after the filter disconnection procedures.

The bag is housed in a metal container called TOTE preventing possible damages of the bag during transportation.

The liposomal formulate is introduced in the sterile bag through the passage on sterilizing filter. The remaining buffer too is sterilized by filtration and added in the bag until obtaining the required weight.

### BULK collection and analysis

On the day after filtration one of the two satellites is filled-up and the sample for the physical and microbiological analyses is collected.

The bulk is sent, in quarantine and provided with the certificate of chemical/physical analyses, to the workshop performing the division activities.

### Division process in asepsis and packaging

The sterile bulk to be divided will be delivered in a pallet-tank (Tote) made of stainless steel, PALL brand.

Tote will include the multi-layered bag of "Allegro" (PALL) system with the product to be divided and it will be equipped with:
Nr. 1 satellite small bag for collecting the sample for the analyses;
Nr. 1 sterile junction.

Omisan, upon receiving the bulk, will provide:
- to verify the presence of certificates of analyses or substitutive authorization by the client (relatively to the microbiological certificate);
- to fill-in the satellite small bag (bulk sample);
- to collect the satellite small bag;
- to perform the chemical and microbiological analyses (sterility test) on the bulk.

The bulk analysis certificates will be enclosed to the Batch record.

If the analysis result is negative the bulk will be destroyed.

The product division in asepsis can be performed even before the result of the analyses on the bulk. In this case, should the analyses on the bulk have negative result, the whole batch will be destroyed. The bulk should be in any case filled-in within 15 days from its receipt date.

The final phase provides the packaging of the solution divided into Novelia flacons without preservatives.

### 3. CLINICAL DATA: EVALUATION OF EFFECTS ON DRY EYE DISEASE (DED) IN PATIENTS UNDERGOING CATARACT SURGERY.

### 3.1 MATERIALS AND METHODS

The study design included four visits: 2 weeks prior to the planned cataract surgery (screening visit), on the day of cataract surgery (baseline visit) and then at week 1 and 2 after surgery (post-surgery visits 1 and 2, respectively). Patients were enrolled in the study at the screening visit, and randomized with a 1:1 ratio to one of the two groups of 23 patients each: group A (experimental group), treated with the ophthalmic composition of the present invention (according to the embodiment of example 1) administered three times a day (TID) starting 2 weeks before surgery until 2 weeks post-surgery for a total of 4 weeks; group B (control group). Both groups received, 3 days before surgery, a standard treatment with ofloxacin and, soon after surgery, a standard postoperative treatment (topical dexamethasone for 10 days combined to ofloxacin for 7 days). Patients were allowed to carry on any systemic or local treatment for possible concomitant diseases.

Topical ophthalmic medications, including lubricating eye drops, were not administered at least 4 days before screening visit for all patients. Major inclusion criteria encompassed adults of both genders diagnosed with cataract requiring surgery, with either healthy ocular surface or mild DED (including subclinical DED) with *tear break-up time* (TBUT) > 7 and Schirmer test> 15 mm/5 minutes. Subjects were excluded from study if affected from neuropathic DED, proven or suspected glaucoma, Sjögren syndrome and/or other autoimmune diseases, corneal diseases, surface eye disturbances and/or other functional and anatomic eyelid abnormalities.

The primary objective of the study was the comparison of the change over time in TBUT between the two treatment groups. The following secondary objectives were also explored: the performances of the ophthalmic composition according to the present invention in reducing changes in ocular surface staining; the changes of Schirmer I test and osmolimetry; the ocular discomfort symptoms, measured by means of a validated questionnaire (OSDI).

### 3.2 RESULTS AND DISCUSSION

Forty-six patients were enrolled in the study and 45 completed the study, 23 into group A and 22 into group B. One patient withdrew her consent before the cataract surgery and was excluded from the analysis. The two groups were well matched for gender, ethnicity, age, height, weight, time elapsed from cataract diagnosis and no statistically significant difference was observed. The demographic results are described in Table 1. The results of the study are summarized in Table 2. At the screening visit, the TBUT values were similar in group A and in group B (8.5 ± 1.8 and 7.8 ± 0.7 seconds respectively, p = 0.11). At baseline prior to the cataract surgery (visit 0), the mean TBUT value in group A was comparable to the one at the screening visit, while a decrease of 0.6 seconds was observer in group B. The difference between the two groups was statistically significant (p = 0.01) (Figure 1). One week after surgery (visit 1) a decrease in TBUT was observed in group A (7.4 ± 1.5 sec) compared to previous visits, while in group B it decreased to 6.0 ± 1.3 sec (p = 0.002). At visit 1, TBUT values were worse than at the screening visit in 57% of patients in group A and in 91% in group B. Two weeks after surgery (visit 2), TBUT showed a trend to return toward the initial mean value observed at the screening visit in group A, while it further decreased in group B (p = 0.0002). The difference between the two groups further increased compared to the visit 1 (Figure 1). Comparing TBUT at the beginning and at the end of the study, a worsening is observed in 57% of patients in group A and in 82% in group B (Table 3).

When the patients were stratified into four classes according to severity of TBUT (e.g. <5 sec, 5-7 sec, 8-9 sec, ≥10 sec), similar distributions were observed in the two groups at screening phase and a progressive advantage of group A over group B was confirmed in the following visits (Table 4). The mean OSDI Questionnaire scores of both study groups were similar at the screening visit. On the day of surgery, a decrease in both groups was observed; this reduction was significantly larger in group A compared with group B (p = 0.01). During the two post-surgery visits, a small increase in *ocular surface disease index (*OSDI) scores was observed in group A, while a greater increase was recorded in group B (Figure 2). The differences in mean OSDI scores between the groups were statistically significant at both visits 1 and 2 (p = 0.01 and p = 0.027, respectively).

Osmolimetry was similar in the two groups at screening and no statistically significant difference was observed between the two groups (Table 2). Moreover, the mean values of Schirmer test were similar between the groups throughout the duration of the study (Table 2).

The results of this clinical study demonstrate that the administration of the present invention is capable to significantly reduce postoperative DED-related signs and symptoms to almost normal values in elderly subjects undergoing cataract surgery. The clinical value is even more evident by comparing it with the group of patients receiving standard postoperative care and whose decline of the ocular surface health retrace the natural prognosis of individuals in which the occurrences of a postoperative DED is underestimated.

**Table 1. Demographic results (ITT-population)**

| | **Group A (n=23)** | **Group B (n=22)** | **Total (n=45)** |
|---|---|---|---|
| **Gender N (%)** | | | |
| Male | 7 (30.4%) | 8 (36.4%) | 15 (33.3%) |
| Female | 16 (69.6%) | 14 (63.6%) | 30 (66.7%) |

| **Ethnic Group** | | | |
|---|---|---|---|
| Caucasian | 23 | 22 | 45 |

| **Age (years)** | | | |
|---|---|---|---|
| Mean ± SD | 73 ± 7 | 76 ± 8 | 74 ± 8 |
| Median (range) | 72 (52-88) | 77 (54-86) | 76 (52-88) |

| Height (cm) | | | |
|---|---|---|---|
| Mean ± SD | 163 ± 10 | 163 ± 8 | 163 ± 9 |
| Median (range) | 164 (146-180) | 160 (140-175) | 162 (140-180) |

| **Weight (kg)** | | | |
|---|---|---|---|
| Mean ± SD | 69 ± 16 | 71 ± 13 | 70 ± 14 |
| Median (range) | 68 (45-95) | 68 (45-100) | 68 (45-100) |

| **Time elapsed from cataract diagnosis (days)*** | | | |
|---|---|---|---|
| Mean ± SD | 600 ± 291 | 763 ± 321 | 679 ± 314 |
| Median | 732 | 765 | 740 |

| | | | |
|---|---|---|---|
| * Time elapsed from cataract diagnosis was calculated as (Cataract diagnosis date -Visit -1) +1 | | | |

**Table 2. Primary and secondary efficacy variables at each study visit (ITT population)**

| **Variables at each study visit** | **Group A (n=23)** | **Group B (n=22)** | ***p*-value** |
|---|---|---|---|
| **TBUT - mean ± SD** | | | |
| Visit -1 | 8.5 ± 1.8 | 7.8 ± 0.7 | 0.11 |
| Visit 0 | 8.4 ± 1.8 | 7.2 ± 0.9 | 0.01 |
| Visit 1 | 7.4 ± 1.5 | 6.0 ± 1.3 | 0.002 |
| Visit 2 | 7.9 ± 1.6 | 6.0 ± 1.5 | 0.0002 |

| **OSDI Questionnaire Score - mean ± SD** | | | |
|---|---|---|---|
| Visit -1 | 13 ± 8 | 14 ± 8 | 0.55 |
| Visit 0 | 6 ± 5 | 11 ± 7 | 0.01 |
| Visit 1 | 8 ± 7 | 16 ± 12 | 0.014 |
| Visit 2 | 9 ± 8 | 16 ± 11 | 0.027 |

| **Osmolimetry Test - mean ± SD** | | | |
|---|---|---|---|
| Visit -1 | 305 ± 17 | 303 ± 13 | 0.77 |
| Visit 0 | 306 ± 17 | 299 ± 17 | 0.21 |
| Visit 1 | 302 ± 18 | 304 ± 17 | 0.79 |
| Visit 2 | 304 ± 16 | 302 ± 13 | 0.66 |

| **Schirmer I test - mean ± SD** | | | |
|---|---|---|---|
| Visit -1 | 20 ± 6 | 18 ± 5 | 0.31 |
| Visit 0 | 20 ± 7 | 16 ± 5 | 0.08 |
| Visit 1 | 16 ± 6 | 16 ± 8 | 0.84 |
| Visit 2 | 18 ± 8 | 16 ± 7 | 0.52 |

| **Staining Grade with Fluorescein - N (%)** | | | |
|---|---|---|---|
| Visit 1 | | | |
| 0 | 18 (78.3%) | 13 (59.1%) | |
| 1 | 4 (17.4%) | 5 (22.7%) | |
| 2 | 1 (4.3%) | 4 (18.2%) | |
| Visit 2 | | | |
| 0 | 15 (65.2%) | 12 (54.5%) | |
| 1 | 7 (30.4%) | 5 (22.7%) | |
| 2 | 1 (4.3%) | 5 (22.7%) | |

| | | | |
|---|---|---|---|
| Visit -1 = screening; Visit 0 = baseline; Visit 1 = week 1; Visit 2 = week 2 | | | |

**Table 3. Stratification of patients of both treatment groups in classes of TBUT severity**

| **TBUT classes** | **Visit -1** | | | **Visit 0** | | | **Visit 1** | | | **Visit 2** | | |
|---|---|---|---|---|---|---|---|---|---|---|---|---|
| | **Group A (n=23) N (%)** | **Group B (n=22) N (%)** | ***p**** | **Group A (n=23) N (%)** | **Group B (n=22) N (%)** | ***p**** | **Group A (n=23) N (%)** | **Group B (n=22) N (%)** | ***p**** | **Group A (n=23) N (%)** | **Group B (n=22) N (%)** | ***p**** |
| <5 sec | 0 (0.00) | 0 (0.00) | 0.17 | 0 (0.00) | 0 (0.00) | 0.02 | 0 (0.00) | 4 (18.2) | 0.01 | 0 (0.00) | 3 (13.6) | 0.004 |
| 5-7 sec | 6 (26.1) | 7 (31.8) | | 8 (34.8) | 14 (63.6) | | 12 (52.2) | 15 (68.2) | | 11 (47.8) | 17 (77.3) | |
| 8-9 sec | 13 (56.5) | 15 (68.2) | | 9 (39.1) | 8 (36.4) | | 10 (43.5) | 3 (13.6) | | 7 (30.4) | 2 (9.1) | |
| ≥10 sec | 4 (17.4) | 0 (0.00) | | 6 (26.1) | 0 (0.00) | | 1 (4.3) | 0 (0.00) | | 5 (21.8) | 0 (0.00) | |

| | | | | | | | | | | | | |
|---|---|---|---|---|---|---|---|---|---|---|---|---|
| * Fisher *p*-value. | | | | | | | | | | | | |

**Table 4. Number and percentage of patients improved, stable and worsened in TBUT values during study visits compared to screening visit (visit -1) by treatment group (ITT population)**

| | **Group A** | | | **Group B** | | |
|---|---|---|---|---|---|---|
| | **N (%)** | | | **N (%)** | | |
| | **Improved** | **Stable** | **Worsened** | **Improved** | **Stable** | **Worsened** |
| Visit -1 vs Visit 0 | 5 (22) | 8 (35) | 10 (43) | 3 (14) | 10 (45) | 9 (41) |
| Visit -1 vs Visit 1 | 2 (9) | 8 (35) | 13 (57) | 0(0) | 2 (9) | 20 (91) |
| Visit -1 vs Visit 2 | 6 (26) | 4 (17) | 13 (57) | 1 (5) | 3 (14) | 18 (82) |

| | | | | | | |
|---|---|---|---|---|---|---|
| Improved = difference between two TBUT values> 0 Stable = difference between two TBUT values = 0 Worsened = difference between two TBUT values <0 | | | | | | |

### 4. CLINICAL DATA: EVALUATION OF THE EFFECTS IN DIFFERENT CLASSES OF PATIENTS WITH DED

### 4.1 Study summary

An additional clinical study was performed to investigate the effectiveness of the ophthalmic composition of the present invention, in particular according to the embodiment of example 1 (designated in the study as MAF1217). The evaluation was made both in absolute and comparative terms by using Cationorm^{®} as control. Cationorm^{®} is a commercially available eye drop comprising: mineral oils, glycerol, tyloxapol, poloxamer 188, tris-hydrochloride, tromethamine, cetalkonium chloride, water for injectable preparations. The study was performed on four types of patients affected by dry eye Syndrome (groups A-D), in particular patients with high levels of evaporation, menopause women, independently from the use of the hormone integration, patients with active obstructive Meibomian gland dysfunction and glaucomatous patients. Several clinical parameters were measured; the primary parameter was TBUT change, while the secondary parameters were: staining of the ocular surface, Schirmer test, number of blinking per minute, ferning test, osmolimetry, collection of the tear film and analysis of the expression of cytokines and lipids, OSDI, product safety and satisfaction level. The study results showed that the treatment with MAF1217 can be compared to that with Cationorm in terms of effectiveness and safety for the treatment of different types of Dry eye. Moreover, the number of complete blinking increases, the number of incomplete blinking and the results obtained through Ferning Test improve with respect to Cationorm control. These data suggest that the composition according to the present invention has even a better protecting and restoring action of the lipidic and mucous layer of the tear film with respect to the used control.

### 4.2. Materials and methods

The described study is of multicentre, randomized, double-blind, cross-over type not inferior than a lipidic tear substitute, Cationorm, which constitutes one of the reference products at European levels.

The study was performed on 72 patients with different dry eye types (hyper evaporative forms, on hormonal basis, alterations of tear secretion of Meibomian glands, glaucomatous patients with damage caused by preservative). The patients had used the product, the present patent application relates to, for 6 weeks and Cationorm for further 6 weeks in randomized sequence, three times a day.

The following clinical parameters were measured: BUT, staining of the ocular surface, Schirmer test, number of blinking per minute, ferning test, osmolimetry, collection of the tear film and analysis of the expression of cytokines and lipids, OSDI. The product safety and satisfying level were measured.

The study included 5 visits: baseline and 2 visits for each period (week 2 and week 6).

### 4.3 Results and discussion

The differences of the primary parameter, TBUT, were not significative in the two groups: baseline 3.2±1.5 sec; visits of week 2 of each period, 4.5±1.9 and 4.5±1.8 sec respectively in the groups of the patent product and Cationorm (p=0.63); final visits respectively 5.4±2.4 and 6.0±3.1 sec (p=0.10). As a consequence, the study product resulted not inferior than Cationorm.

For the secondary parameters a significative difference between the two products was not verified, except for the decrease in the number of blinking, higher for MAF-1217, and the number of patients with normal ferning test at the end of the study period, higher for MAF-1217 with respect to Cationorm. The two products had obtained the same results in terms of reduction of symptoms (OSDI questionnaire). The safety profile of both products was excellent. Even the blurred vision feeling after administration was superimposable in the two groups.

In conclusion, this not-inferiority study confirmed that the clinical effectiveness of the product, the present patent application relates to, is comparable to that of the clinical reference between the lipidic tear substitutes, that is Cationorm.

The two parameters favourable to the product, the present patent application relates to, (decrease in the total number of blinking per minute and ferning test) seem to suggest that MAF-1217 is capable to restore a normal health of the ocular surface better than Cationorm.

**Table 5. Demographic features and different sub-groups of DED in groups of sequence A and sequence B.**

| | **Sequence A* (n=37)** | **Sequence B** (n=26)** | **Total (n=63)** |
|---|---|---|---|
| **Gender - N (%)** | | | |
| Male | 11 (30%) | 6 (23%) | 17 (27%) |
| Female | 26 (70%) | 20 (77%) | 46 (73%) |

| **Age (years)** | | | |
|---|---|---|---|
| Mean ± SD | 65 ± 17 | 64 ± 16 | 65 ± 17 |
| Median (range) | 69 (21-89) | 68 (25-91) | 69 (21-91) |

| **Height (cm)** | | | |
|---|---|---|---|
| Mean ± SD | 162 ± 14 | 161 ± 12 | 161 ± 13 |
| Median (range) | 164 (110-183) | 161 (110-175) | 162 (110-183) |

| **Weight (kg)** | | | |
|---|---|---|---|
| Mean ± SD | 71 ± 13 | 73 ± 13 | 71 ± 13 |
| Median (range) | 70 (49-100) | 70 (55-104) | 70 (49-104) |

| **Diagnosis of DED - N (%)** | | | |
|---|---|---|---|
| evaporative DED | 9 (24%) | 7 (27%) | 16 (25%) |
| Women with hormonal changes (menopause)) | 10 (27%) | 5 (19%) | 15 (24%) |
| Obstructive meibomitis in active phase | 10 (27%) | 8 (31%) | 18 (29%) |
| Patients with glaucoma | 8 (22%) | 6 (23%) | 14 (22%) |

| **Previous treatments for DED - N (%)** | | | |
|---|---|---|---|
| Naïve subjects | 18 (49%) | 11 (42%) | 29 (46%) |
| Previously treated subjects | 19 (51%) | 15 (58%) | 34 (54%) |

| | | | |
|---|---|---|---|
| **** Sequence A:*** VisuEvo^{®} during the first 6 weeks, Cationorm^{®} during the subsequent 6 weeks. ****** ***Sequence B:*** Cationorm^{®} during the first 6 weeks, VisuEvo^{®} during the subsequent 6 weeks. | | | |

**Table 6. Results (median values and ranges) of primary and secondary endpoints.**

| **Parameters** | **Median (range)** | |
|---|---|---|
| | **VisuEvo^{®}** | **Cationorm^{®}** |
| **TBUT (sec)** | | |
| Baseline | 3.0 (0.0-6.0) | 3.0 (0.0-6.0) |
| Intermediate visit | 4.0 (0.0-10.0) | 4.0 (0.0-10.0) |
| Final visit | 5.0 (2.0-11.0) | 6.0 (1.0-14.0) |
| **Schirmer I test (mm/5 min)** | | |
| Baseline | 14 (11-31) | 14 (11-31) |
| Intermediate visit | 13 (2-35) | 13 (2-35) |
| Final visit | 12 (2-35) | 12 (2-35) |
| **Complete blinking (N/min)** | | |
| Baseline | 10 (3-39) | 10 (3-39) |
| Intermediate visit | 18 (0-39) | 18 (0-39) |
| Final visit | 11 (0-54) | 12 (1-81) |
| **Incomplete blinking (N/min)** | | |
| Baseline | 6 (0-27) | 6 (0-27) |
| Intermediate visit | 4 (0-20) | 4 (0-20) |
| Final visit | 3 (0-18) | 1 (0-6) |
| **Total blinking (N/min)** | | |
| Baseline | 22 (3-41) | 22 (3-41) |
| Intermediate visit | 20 (2-59) | 20 (2-59) |
| Final visit | 19 (4-57) | 17 (2-82) |
| **Osmolimetry (mOsm/L)** | | |
| Baseline | 313 (275-353) | 313 (275-353) |
| Intermediate visit | 305 (281-332) | 308 (277-329) |
| Final visit | 305 (280-341) | 306 (278-344) |
| **IL-6 (pg/ml)** | | |
| Baseline | 6.53 (1.18-59.79) | 6.53 (1.18-59.79) |
| Final visit | 4.20 (1.11-53.20) | 4.52 (1.20-29.30) |
| **IL-8 (pg/ml)** | | |
| Baseline | 388 (76-2792) | 388 (76-2792) |
| Final visit | 216 (46-7670) | 235 (79-2268) |
| **IFN (pg/ml)** | | |
| Baseline | 2.54 (0.10-9.04) | 2.54 (0.10-9.04) |
| Final visit | 1.24 (0.10-10.17) | 1.00 (0.10-4.85) |
| **OSDI questionnaire score** | | |
| Baseline | 35 (19-80) | 35 (19-80) |
| Intermediate visit | 21 (0-57) | 21 (0-57) |
| Final visit | 20 (0-55) | 18 (0-64) |

**Table 7. Type, number, severity and relation to the respective ophthalmic treatment of adverse events (AEs) observed during the study.**

| **AEs type** | **VisuEvo^{®} (N° AEs)** | **Cationorm^{®} (N° AEs)** | **Severity** | **Relation to treatment** |
|---|---|---|---|---|
| Bilateral ocular burning | 2 | | Mild | Definitive/none |
| Ocular burning | | 1 | Moderate | Probable |
| Right leg sciatica | 1 | | Moderate | None |
| Non-Hodgkin Lymphoma | 1 | | Severe | None |
| Right eye stinging pain | 1 | | Mild | None |
| Foreign body feeling in right eye | | 1 | Mild | Possible |
| Lymph node biopsy | 1 | | Moderate | None |
| Viral conjunctivitis | 1 | | Severe | None |
| Viral conjunctivitis | | 1 | Moderate | None |
| Suspected conjunctivitis | 1 | | Mild | None |
| Cataract surgery | | 1 | Mild | None |
| Blurred vision* | 11 | 10 | | |
| | 16 | | | |
| **Total** | **8** | **4** | | |

| | | | | |
|---|---|---|---|---|
| * Some patients had blurred vision with one treatment only, while others with both of them. | | | | |

## Claims

1. An ophthalmic composition comprising omega-3 fatty acids, Vitamin A or esters thereof, Vitamin D3 and liposomes.

2. The composition according to claim 1 comprising Vitamin A palmitate.

3. The composition according to claim 1 or 2, wherein said Omega 3 fatty Acids are in an amount comprised between 0.01 and 2% w/w.

4. The composition according to any one of claims 1 to 3, wherein said Vitamin A palmitate is in an amount comprised between 0.01 and 2% w/w.

5. The composition according to any one of claims 1 to 4, wherein said Vitamin D3 is in an amount comprised between 0.01 and 2% w/w.

6. The composition according to any one of claims 1 to 4, wherein said Liposomes are in an amount comprised between 0.5 and 4% w/w.

7. The composition according to any one of claims 1 to 6 wherein said liposomes comprise or consist of a double layer of phospholipids and an aqueous nucleus.

8. The composition according to any one of claims 1 to 7 in a form selected among solution, ointment, suspension, eye drop, gel, cream, foam, spray, pomade.

9. The composition according to any one of claims 1 to 8 wherein said liposomes have a size comprised between 75 and 300 nm.

10. The composition according to any one of claims 1 a 9, wherein said omega 3 fatty acids are oils extracted from marine microalga marina *Schizochytrium sp.*

11. The composition according to any one of claims 1 to 10, wherein said omega 3 fatty acids are eicosapentaenoic acid (EPA), docosapentaenoic acid (DPA) and/or docosaesaenoic acid (DHA).

12. The composition according to any one of claims 1 to 11 for use in the prevention and/or treatment of dry eye disease.

13. The composition according to any one of claims 1 a 11 for use in the prevention and/or treatment of dry eye disease associated to a cataract surgery.

14. The composition according to any one of claims 1 a 11 for use in the prevention and/or treatment of dry eye disease in a patient with glaucoma.

## Patentansprüche

1. Ein ophthalmisches Stoffgemisch umfassend Omega-3-Fettsäuren, Vitamin A oder Ester davon, Vitamin D3 und Liposomen.

2. Stoffgemisch gemäß Anspruch 1 umfasst Vitamin A-Palmitat.

3. Stoffgemisch gemäß Anspruch 1 oder 2, wobei die Omega-3-Fettsäuren in einer Menge zwischen 0,01 und 2 Gewichts-% enthalten sind.

4. Stoffgemisch gemäß einem der Ansprüche 1 bis 3, wobei das Vitamin A-Palmitat in einer Menge zwischen 0,01 und 2 Gewichts-% enthalten ist.

5. Stoffgemisch gemäß einem der Ansprüche 1 bis 4, wobei das Vitamin D3 in einer Menge zwischen 0,01 und 2 Gewichts-% enthalten ist.

6. Stoffgemisch gemäß einem der Ansprüche 1 bis 4, wobei die Liposomen in einer Menge zwischen 0,5 und 4 Gewichts-% enthalten sind.

7. Stoffgemisch gemäß einem der Ansprüche 1 bis 6, wobei die Liposomen eine Doppelschicht aus Phospholipiden und einen wässrigen Kern umfassen oder daraus bestehen.

8. Stoffgemisch gemäß einem der Ansprüche 1 bis 7 in einer Form, ausgewählt aus Lösung, Salbe, Suspension, Augentropfen, Gel, Creme, Schaum, Spray, Pomade.

9. Stoffgemisch gemäß einem der Ansprüche 1 bis 8, wobei die Liposomen eine Größe haben, die zwischen 75 und 300 nm liegt.

10. Stoffgemisch gemäß einem der Ansprüche 1 bis 9, wobei die Omega-3-Fettsäuren Öle sind, die aus der Meeresmikroalge *Schizochytrium sp.* extrahiert wurden.

11. Stoffgemisch gemäß einem der Ansprüche 1 bis 10, wobei die Omega-3-Fettsäuren Eicosapentaensäure (EPA), Docosapentaensäure (DPA) und/oder Docosaensäure (DHA) sind.

12. Stoffgemisch gemäß einem der Ansprüche 1 bis 11 zur Verwendung bei der Vorbeugung und/oder Behandlung des trockenen Auges.

13. Stoffgemisch gemäß einem der Ansprüche 1 bis 11 zur Verwendung bei der Vorbeugung und/oder Behandlung des trockenen Auges im Zusammenhang mit einer Kataraktoperation.

14. Stoffgemisch gemäß einem der Ansprüche 1 bis 11 zur Verwendung bei der Vorbeugung und/oder Behandlung des trockenen Auges bei einem Patienten mit einem Glaukom.

## Revendications

1. Composition à usage ophtalmique comprenant des acides gras oméga-3, de la vitamine A ou des esters de celle-ci, de la vitamine D3 et des liposomes.

2. Composition selon la revendication 1, comprenant du palmitate de vitamine A.

3. Composition selon la revendication 1 ou 2, dans laquelle lesdits acides gras oméga 3 sont présents en une quantité comprise entre 0,01 et 2 % en poids/poids.

4. Composition selon l'une quelconque des revendications 1 à 3, dans laquelle ledit palmitate de vitamine A est présent en une quantité comprise entre 0,01 et 2 % en poids/poids.

5. Composition selon l'une quelconque des revendications 1 à 4, dans laquelle ladite vitamine D3 est présente en une quantité comprise entre 0,01 et 2 % en poids/poids.

6. Composition selon l'une quelconque des revendications 1 à 4, dans laquelle lesdits liposomes sont présents en une quantité comprise entre 0,5 et 4 % en poids/poids.

7. Composition selon l'une quelconque des revendications 1 à 6, dans laquelle lesdits liposomes comprennent ou consistent en une double couche de phospholipides et une âme aqueuse.

8. Composition selon l'une quelconque des revendications 1 à 7, sous une forme choisie parmi une solution, un onguent, une suspension, une goutte oculaire, un gel, une crème, une mousse, un spray, une pommade.

9. Composition selon l'une quelconque des revendications 1 à 8, dans laquelle lesdits liposomes ont une taille comprise entre 75 et 300 nm.

10. Composition selon l'une quelconque des revendications 1 à 9, dans laquelle lesdits acides gras oméga 3 sont des huiles extraites de microalgues marines *Schizochytrium sp.*

11. Composition selon l'une quelconque des revendications 1 à 10, dans laquelle lesdits acides gras oméga 3 sont l'acide eicosapentaénoïque (EPA), l'acide docosapentaénoïque (DPA) et/ou l'acide docosahexaénoïque (DHA).

12. Composition selon l'une quelconque des revendications 1 à 11, destinée à être utilisée dans la prévention et/ou le traitement d'une sécheresse oculaire.

13. Composition selon l'une quelconque des revendications 1 à 11, destinée à être utilisée dans la prévention et/ou le traitement d'une sécheresse oculaire associée à une opération chirurgicale de la cataracte.

14. Composition selon l'une quelconque des revendications 1 à 11, destinée à être utilisée dans la prévention et/ou le traitement d'une sécheresse oculaire chez un patient présentant un glaucome.
